# FASCICULE DE BREVET EUROPEEN

(11) **EP 1 041 997 B1**
(45) Date de publication et mention de la délivrance du brevet: **16.04.2003**
(21) Numéro de dépôt: 98964537.9
(22) Date de dépôt: 29.12.1998
(51) Int. Cl.: A61K 38/17, C07K 14/47, A61P 37/00

(54) **UTILISATION DE PEPTIDES CITRULLINES DERIVES DE LA FILAGGRINE DANS LE CADRE DU TRAITEMENT DE MALADIES AUTOIMMUNES**
VERWENDUNG VON CITRULLIN ENTHALTENDEN PEPTIDEN, DIE VON FILAGGRIN ABSTAMMEN, ZUR BEHANDLUNG VON AUTOIMMUNKRANKHEITEN
USE OF CITRULLINE PEPTIDES DERIVED FROM FILAGGRIN FOR TREATING AUTOIMMUNE DISEASES

(30) Priorité: 30.12.1997 FR 9716672
(43) Date de publication de la demande: 11.10.2000
(73) Titulaire: UNIVERSITE PAUL SABATIER (TOULOUSE III), F-31062 Toulouse Cédex (FR)
(72) Inventeur: SERRE, Guy, Résidence du Lac, F-31100 Toulouse (FR); GIRBAL-NEUHAUSER, Elisabeth, F-31000 Toulouse (FR); VINCENT, Christian, F-31650 Lauzerville (FR); SIMON, Michel, F-31450 Belberaud (FR); SEBBAG, Mireille, F-31500 Toulouse (FR); DALBON, Pascal, F-69006 Lyon (FR); JOLIVET-REYNAUD, Colette, F-69500 Bron (FR); ARNAUD, Michel, F-69100 Villeurbanne (FR); JOLIVET, Michel, F-69500 Bron (FR)
(74) Mandataire: Vialle-Presles, Marie José
(86) Numéro de dépôt international: FR9802900
(87) Numéro de publication internationale: WO99034819

(56) Documents cités:
- EP-A- 0 511 116
- WO-A-91/11718
- WO-A-94/17411
- WO-A-98/08946
- WO-A-98/22503
- WO-A-99/28344
- WO-A-99/35167
- VII AND VIII ANNUAL MEETINGS OF THE ASSOCIATION PUOR LA RECHERCHE SUR LA POLYARTHRITE,octobre 1996 (1996-10) - octobre 1997 (1997-10), Paris -& GIRBAL-NEUHAUSER E. ET AL.: "The epitopes targeted by the rheumatoid arthritis-associated antifilaggrin autoantibodies are posttranslationally generated on various sites of (pro)filaggrin by deimination of arginine residues" J.IMMUNOL., vol. 162, no. 1, 1 janvier 1999 (1999-01-01), pages 585-594, XP002108878 United States
- DURIEUX J-J. ET AL.: "Identification of two major epitopes targetted by the rheumatoid arthritis -specific anti-filaggrin auto-antibodies" REVUE DU RHUMATISME ENGLISH EDITION, vol. 10, octobre 1997 (1997-10), page 597 XP002108876
- SCHELLEKENS G A ET AL: "THE MODIFIED ARGININE RESIDUE CITRULLINES IS THE MAJOR CONSTITUENT OF EPITOPES RECOGNIZED BY AUTOANTIBODIES IN SERA FROM RHEUMATOID ARTHRITIS PATIENTS" ARTHRITIS & RHEUMATISM, vol. 40, no. 9, SUPPL. 08, 8 novembre 1997 (1997-11-08), page S276 XP002067877 cité dans la demande
- GAN S. ET AL.: "Organization, structure, and polymorphisms of the human profilaggrin gene" BIOCHEMISTRY, vol. 29, 1990, pages 9432-9440, XP002030710
- SCHELLEKENS GG ET AL.: "Citrulline is an essential constituent of antigenic determinant recognized by rheumatoid arthritis-specific autoantibodies" J.CLIN.INVEST., vol. 101, no. 1, 1 janvier 1998 (1998-01-01), pages 273-81, XP002108877

## Description

La présente invention est relative à l'utilisation de peptides dérivés de la filaggrine dans le traitement de maladies autoimmunes, notamment de la polyarthrite rhumatoïde (PR).

Des anticorps circulants ont été détectés dans le sang de patients atteints de polyarthrite rhumatoïde, et proposés comme marqueurs spécifiques de cette maladie.

Ces anticorps ont été initialement répartis en 2 familles :
- les facteurs antipérinucléaires (APF) qui sont des immunoglobulines circulantes, détectables par immunofluorescence indirecte sur frottis de cellules jugales humaines ; leurs performances diagnostiques moyennes dans le diagnostic de la PR sont d'environ 90 % de spécificité pour une sensibilité de 70 %.
- les anticorps dénommés "antikératine" (AKA) qui sont des immunoglobulines G circulantes détectables par immunofluorescence sur cryocoupes d'oesophages de rat. Ces anticorps sont très spécifiques de la polyarthrite rhumatoïde et leurs performances diagnostiques moyennes sont de 98 % de spécificité pour une sensibilité d'environ 50 %.

Des travaux antérieurs de l'équipe des Inventeurs ont abouti à la caractérisation des antigènes reconnus par ces anticorps, et ont ainsi permis de , montrer que ceux-ci constituaient en fait une seule et même famille d'auto-anticorps, et qu'ils étaient dirigés contre différentes formes moléculaires de la famille des (pro)filaggrines (pour revue, cf. par exemple SERRE et VINCENT, In : Autoantibodies, PETER and SHOENFELD Eds, Elsevier Science Publishers, 271-276, 1996). Ces anticorps ont été dénommés auto-anticorps anti-filaggrine (AAF) ;. La Demande EP 0 511 116 décrit la purification et la caractérisation d'antigènes de la famille des filaggrines reconnus par ces anticorps, et leur utilisation pour le diagnostic de la polyarthrite rhumatoïde.

En poursuivant leurs travaux, les Inventeurs ont montré que les épitopes reconnus par ces anticorps étaient portés par des régions de la molécule de filaggrine dans lesquelles au moins une partie des arginines étaient déiminées, et transformées de la sorte en citrulline ; ils ont ainsi identifié les principales régions immunoréactives, et ont ensuite obtenu à partir de celles-ci des peptides portant des épitopes intégrant un résidu citrulline, spécifiquement reconnus par les auto-anticorps anti-filaggrine. Ces peptides, et leur utilisation pour le diagnostic de la PR font l'objet de la demande FR 96 10651, au nom de BIOMERIEUX, déposée le 30 août 1996, et de la demande PCT/FR 97/01541, au nom de BIOMERIEUX, déposée le 1^{er} septembre 1997. Les observations des Inventeurs concernant le rôle de résidus citrulline dans la réactivité de la filaggrine avec les auto-anticorps spécifiques de la PR ont été ultérieurement confirmées par d'autres chercheurs [SCHELLEKENS et al., Arthritis Rheum., 40, n° 9 supplément, p. S276, résumé 1471 (1997); VISSER et al., Arthritis Rheum., 40, n° 9 supplément, p. S289, résumé 1551 (1997)]. La Demande PCT WO 99/28344 qui appartient à l'art antérieur selon l'article 54(3) CBE, décrit différents peptides citrullinés reconnus par ces auto-anticorps.

Les Inventeurs ont maintenant entrepris d'étudier le rôle joué par la réponse immunitaire vis-à-vis de ces épitopes citrullinés, dans la genèse de la PR.

Dans ce but, ils ont comparé le rapport, dans le sérum et dans les tissus rhumatoïdes, entre les auto-anticorps anti-filaggrine de classe G spécifiquement dirigés contre ces épitopes, et les IgG totales et ont observé que ce rapport était environ 10 fois plus élevé dans les immunoglobulines interstitielles du tissu rhumatoïde, que dans les immunoglobulines sériques. Ils ont en outre montré qu'il existait une synthèse localisée, dans les tissus rhumatoïdes, de ces auto-anticorps anti-filaggrine de classe G par des plasmocytes spécifiques présents dans ces tissus.

Ces résultats montrent qu'il existe au niveau des tissus rhumatoïdes une réponse auto-immune locale, dirigée contre des épitopes citrullinés similaires à ceux reconnus par les auto-anticorps anti-filaggrine, et dont ces auto-anticorps sont l'un des constituants. Cette réponse de nature humorale et/ou cellulaire, pourrait être impliquée dans la physiopathogénie rhumatoïde.

Ces observations conduisent à proposer l'utilisation de molécules portant les épitopes citrullinés reconnus par ces auto-anticorps anti-filaggrine pour neutraliser cette réponse auto-immune, et en particulier pour inhiber la fixation à leur antigène-cible des tissus rhumatoïdes, des effecteurs humoraux ou cellulaires de cette réponse auto-immune.

Une telle neutralisation *in vivo* de cette réponse auto-immune, peut participer au traitement de la PR, ou d'autres maladies dans lesquelles interviennent des lésions induites par une réponse auto-immune dirigée contre des épitopes citrullinés présentant des réactions croisées avec ceux reconnus par les anticorps anti-filaggrine.

La présente Invention a pour objet l'utilisation d'un antigène peptidique reconnu spécifiquement par les auto-anticorps anti-filaggrine présents dans le sérum de patients atteints de polyarthrite rhumatoïde, constitué par un peptide dérivé de tout ou partie de la séquence d'une unité filaggrine, dans laquelle au moins un résidu arginine a été substitué par un résidu citrulline, et comprenant au moins un motif tripeptidique Ser-Cit-His, dans lequel Cit représente un résidu citrulline, pour l'obtention d'un médicament inhibiteur destiné au traitement d'une pathologie impliquant une réponse auto-immune vis-à-vis d'épitopes citrullinés reconnus par lesdits anticorps anti-filaggrine, à l'exception de l'utilisation d'un peptide séquence :
Gln Gly Ser Cit His Gln Gln Ala Arg et des peptides comprenant ladite séquence, dérivés de l'un des peptides suivants :
   - Gly Gly Gln Gly Ser Arg His Gln Gln Ala Arg ;
   - Gln Gly Ser Arg His Gln Gln Ala Arg Asp Ser Ser Arg His Ser Thr Ser Gln Glu Gly Gln Asp Thr Ile His Gly His Arg Gly Ser ;
   - Gln Gly Ser Arg His Gln Gln Ala Arg Asp Ser Ser Arg His Ser Ala Ser Gln Asp Gly Gln Asp Thr Ile Arg Gly His Pro Gly Ser Ser ;
   décrits dans la Demande PCT WO 99/28344.

Au sens de la présente Invention, on entend par :
- « séquence d'une unité filaggrine », la séquence du produit de traduction de l'une quelconque des sous-unités du gène de la profilaggrine humaine ou d'une autre espèce animale codant pour un domaine filaggrine, ou bien une séquence consensus théorique, obtenue à partir des séquences des domaines filaggrine.
- « peptide dérivé de tout ou partie de la séquence d'une unité filaggrine »
   a) tout polypeptide ou oligopeptide, comprenant la totalité de ladite séquence ou un fragment de celle-ci, et comprenant au moins un résidu citrulline ; il peut s'agir par exemple d'un fragment de protéolyse de la (pro)filaggrine, ou bien d'un peptide synthétique ou recombinant ;
   b) tout peptide résultant de la modification d'un peptide tel que défini en a) ci-dessus, à condition que cette modification ne détruise pas les propriétés de réaction spécifique avec les auto-anticorps anti-filaggrine.

   Des peptides modifiés utilisables conformément à l'invention sont par exemple des mimotopes de peptides tels que définis en a) ci-dessus.
   Avantageusement, pour l'administration in *vivo,* on utilisera des peptides modifiés de manière à prolonger leur durée de vie dans l'organisme, en particulier en augmentant leur résistance aux protéases ; il peut s'agir par exemple de pseudopeptides de type *rétro*, dans lesquels des acides L-aminés sont enchaînés selon une séquence inverse de celle du peptide à reproduire, ou bien de pseudopeptides de type *rétro-inverso*, constitués par des acides aminés de la série D (au lieu des acides aminés de la série L des peptides naturels) enchaînés selon une séquence inverse de celle du peptide à reproduire, ou bien encore de pseudopeptides contenant une liaison CH₂-NH à la place d'une liaison peptidique CO-NH. Ces pseudopeptides permettent d'imiter la structure tridimensionnelle de peptides naturels, et donc leur réactivité immunologique. Des pseudopeptides de ces différents types sont par exemple décrits par BENKIRANE et al. [J. Biol. Chem., 270, p. 11921-11926, (1995) ; J. Biol. Chem., 271, p. 33218-33224, (1996)] ; BRIAND et al. [J. Biol. Chem., 270, p. 20686-20691, (1995) ; GUICHARD et al. [J. Biol. Chem., 270, p. 26057-26059, (1995)].
- « molécule de reconnaissance de même spécificité que les auto-anticorps anti-filaggrine » : toute molécule (anticorps ou récepteur cellulaire) ayant la propriété de se combiner spécifiquement, *in vivo* ou *in vitro*, avec les épitopes reconnus par les auto-anticorps anti-filaggrine.

Selon un mode de mise en oeuvre préféré de la présente Invention on utilise au moins un peptide citrulliné comprenant tout ou partie d'au moins une séquence dérivée de l'une des régions suivantes d'une unité filaggrine humaine :
- la région correspondant à la portion C-terminale (acides aminés 144 à 324) et en particulier aux acides aminés 144 à 314 d'une unité filaggrine humaine, ou bien
- de la région correspondant aux acides aminés 76 à 144 d'une unité filaggrine humaine, ou bien
- de la région correspondant aux acides aminés 71 à 119 d'une unité de filaggrine humaine.

Ces régions ont été précédemment identifiées (cf. Demande FR 96 10651 et Demande PCT/FR97/01541 mentionnées ci-dessus) comme étant, après citrullination, fortement immunoréactives vis-à-vis des auto-anticorps anti-filaggrine.

Selon un autre mode de mise en oeuvre préféré de la présente invention, on utilisera au moins un peptide citrulliné comprenant :
a) au moins une séquence citrullinée comprenant le motif Ser-Cit-His, dérivée de l'une des séquences représentées dans la liste de séquences en annexe sous les numéros SEQ ID NO: 1, SEQ ID NO: 2, SEQ ID NO: 3, ou bien ;
b) au moins un fragment comprenant le motif Ser-Cit-His, et comprenant au moins 3, de préférence au moins 5, et avantageusement au moins 10 acides aminés consécutifs de ladite séquence.

Des peptides utilisables, conformément à l'invention, pour atténuer ou neutraliser une réponse autoimmune vis-à-vis d'épitopes citrullinés reconnus par des auto-anticorps anti-filaggrine, en réduisant ou en inhibant la fixation à leur antigène-cible, des effecteurs humoraux ou cellulaires de cette réponse autoimmune, sont avantageusement des peptides dérivés, par citrullination, de peptides qui comprennent le motif pentapeptidique, X1-Ser-Arg-His-X2 dans lequel X1=Ser ou Gly, et X2=Ser ou Pro, et parmi ceux-ci, des peptides qui comprennent le motif hexapeptidique X0-X1-Ser-Arg-His-X2, ou le motif heptapeptidique X0-X1-Ser-Arg-His-X2-X3, dans lesquels X1 et X2 sont tels que définis ci-dessus, X0=Asp, et X3=Gly ou Arg.

Pour la mise en oeuvre de la présente invention, on utilisera un peptide comprenant au moins 5 acides aminés, de préférence au moins 10 acides aminés, et avantageusement au moins 14 acides aminés. Avantageusement on peut également utiliser des peptides comprenant plusieurs séquences citrullinées, telles que définies ci-dessus, identiques entre elles ou différentes.

La présente invention englobe également des compositions pharmaceutiques, notamment pour le traitement de la polyarthrite rhumatoïde, caractérisées en ce qu'elles contiennent en tant que principe actif, au moins un peptide antigénique tel que défini ci-dessus.

Des compositions pharmaceutiques conformes à l'invention peuvent être administrées par tous moyens appropriés, connus en eux-mêmes. Elles peuvent par exemple être administrées de manière systémique, par voie orale, ou par voie parentérale, en injection sous-cutanée, intraveineuse ou intramusculaire ; elles peuvent également être administrées localement, par exemple par injections intra-articulaire, ou par micro-injections sous arthroscopie dans le tissu synovial inflammatoire.

La présente Invention sera mieux comprise à l'aide du complément de description qui va suivre, qui se réfère à des exemples démontrant l'accumulation d'auto-anticorps anti-filaggrine de classe G, dans des tissus rhumatoïdes et la synthèse de ces auto-anticorps par des plasmocytes présents dans ces tissus.

### EXEMPLE 1 : COMPARAISON DES CONCENTRATIONS EN ANTICORPS ANTIFILAGGRINE (AAF) DANS LE SERUM ET LE LIQUIDE SYNOVIAL DE PATIENTS ATTEINTS DE POLYARTHRITE RHUMATOIDE.

Des échantillons appariés de sérum et de liquide synovial ont été obtenus à partir de 31 patients adultes atteints de polyarthrite rhumatoïde, diagnostiquée selon les critères cliniques, radiographiques et biologiques de l'AMERICAN RHEUMATISM ASSOCIATION (ARA) (ARNETT AR 31 315-324 1988). Les liquides synoviaux ont été prélevés sur les articulations enflammées puis centrifugés et conservés à -80°C jusqu'au moment de l'analyse.

Les concentrations en IgG totales, et en AAF des sérums et des liquides synoviaux ont été mesurées par ELISA sandwich , selon les protocoles suivants :

### Mesure des IgG totales

Les puits de plaques de microtitration (NUNC IMMONOPLATE MAXISORP) ont été incubés pendant une nuit à 4°C avec 100 µl d'anticorps de chèvre anti-IgG humaine, purifiés par chromatographie d'affinité (BIOSYS), à une concentration de 5 µg par ml dans du PBS.

Les puits revêtus d'anticorps de chèvre ont été incubés directement avec 100 ul d'une série de dilutions de sérum (dilution de 1/400 à 1/1600), ou de liquide synovial (dilution de 1/100 à 1/10000), suivi par 100 µl d'anticorps de chèvre anti-IgG humaine marqué à la peroxydase, et dilué au 1/5000. Pour la quantification des mesures, on a utilisé comme gamme d'étalonnage sur chaque plaque ELISA, une gamme de dilutions (de 500 à 7,8 ng/ml) d'IgG humaines purifiées (JACKSON IMMUNORESEARCH LABORATORIES, West Grove, PE).

Toutes les incubations ont été effectuées pendant une heure à 37°C, et suivies par 3 lavages. Le tampon de dilution et de lavage est du PBS, contenant 0,5% (p/v) de gélatine de poisson et 0,05% (v/v) de Tween-20. L'activité peroxydase a été révélée avec une solution chromogène d'O-phénylènediamine (2 mg/ml, SIGMA)-H₂O₂ (0,03%, SIGMA).

La réaction est stoppée par addition d'H₂SO₄ 4M, et la densité optique à 492 nm est mesurée avec un lecteur de plaques MULTISCAN RC, (LABSYSTEM, Helsinki, Finlande).

### Détermination des auto-anticorps anti-filaggrine.

### Test ELISA sur filaggrine humaine neutre/acide.

Les puits de plaques de microtitration (NUNC IMMUNOPLATE MAXISORP, LIFE TECHNOLOGIES, Paisley, UK) ont été revêtus de filaggrine humaine neutre/acide par incubation pendant une nuit à 4°C avec 100 µl d'une solution de filaggrine humaine neutres/acide à 2,5 mg/ml dans du PBS. Ces puits ont ensuite été traités pendant 30 minutes à 37°C avec 200 µl d'une solution contenant 2,5% (p/v) de gélatine de poisson (SIGMA), et 0,05% (v/v) de Tween-20 (PIERCE) dans du PBS. Chacun des puits revêtus de filaggrine a été incubé avec 100 µl d'une dilution de sérum (1/400 à 1/1600), ou de liquide synovial (1/100 à 1/1600), puis avec 100 µl d'anticorps de chèvre anti-IgG humaine marqués à la peroxydase (SOUTHERN BIOTECHN INC., Birmingham, AL), dilués au 1/2000.

Le temps d'incubation, les tampons utilisés et le système de détection sont identiques à ceux indiqués ci-dessus dans le cas de l'ELISA sur les IgG totales.

Pour quantifier les anticorps anti-filaggrine, on a utilisé un pool rassemblant 13 sérums PR possédant des titres élevés en anticorps anti-filaggrine préalablement évalués par immunofluorescence et immunotransfert. Ce pool a été calibré par rapport à des anticorps anti-filaggrine purifiés. L'immunoréactivité du pool équivalait à une concentration en anticorps anti-filaggrine de 1600 µg par ml. Une série de dilutions successives (1/100 à 1/12800) de ce pool, correspondant à des concentrations de 0,125 à 16 µg par ml d'anticorps anti-filaggrine, a été utilisée sur chaque plaque ELISA, comme gamme étalon pour les échantillons analysés sur la même plaque. La courbe étalon a été linéarisée par log/logit, et utilisée pour traduire les densités optiques des échantillons en concentrations en anticorps anti-filaggrine. Une concentration de l'échantillon dilué inférieure à 0,125 µg/ml (point le plus bas de la courbe étalon) était considérée comme nulle, et pour une concentration supérieure à 4 µg/ml, l'analyte a été essayé à nouveau à dilution plus élevée. Un sérum contrôle de PR a également été ajouté à chaque plaque ELISA, pour apprécier la variabilité inter-essais. Toutes les mesures ont été effectuées au moins en double.

### Résultats

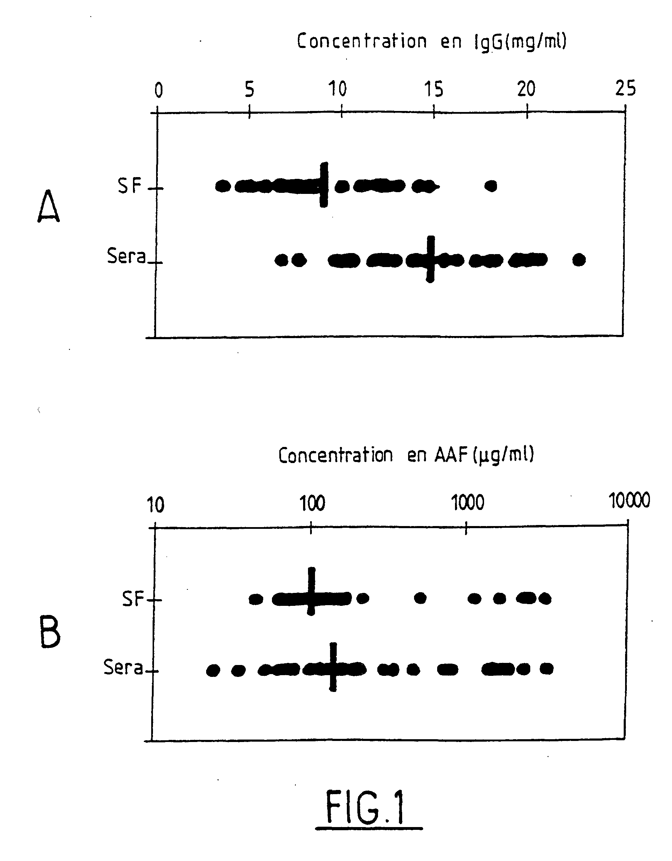

Les résultats sont illustrés par la Figure 1 : (Figure 1 A : IgG ; Figure 1 B : AAF), qui montre que pour tous les patients, la concentration en IgG totales est plus élevée dans le sérum (14,2 ± 0,7 mg/ml) que dans le liquide synovial (8,9 ± 0,8 mg/ml). La valeur du rapport de la concentration en IgG totales dans le sérum par rapport au liquide synovial pour un patient donné varie de 1 à 3.

Pour obtenir une comparaison valable, la concentration en anticorps anti-filaggrine dans le liquide synovial de chaque patient a été évaluée en tenant compte du rapport de la concentration totale en IgG dans le liquide synovial à la concentration totale en IgG dans le sérum du même patient. Les résultats des couples sérum/liquide synovial ont été comparés, et considérés comme différents quand l'immunoréactivité des anticorps anti-filaggrine était 2 fois plus élevée ou 2 fois plus faible dans le liquide synovial que dans le sérum.

Dans ces conditions, les concentrations en anticorps anti-filaggrine étaient similaires, ou très proches, dans le sérum et dans le liquide synovial de l'ensemble des patients, à l'exception de 3 d'entre eux. Pour ces 3 patients, les concentrations en anticorps anti-filaggrine étaient significativement (4 à 16 fois) plus faibles dans le liquide synovial que dans le sérum. Cependant, sur la moyenne des résultats, les concentrations des anticorps anti-filaggrine dans le sérum et dans le liquide synovial ne sont pas significativement différentes. Ceci indique que la proportion d'IgG spécifiques de la filaggrine n'est pas plus importante dans le liquide synovial que dans le sérum.

### EXEMPLE 2 : COMPARAISON DES CONCENTRATIONS EN ANTICORPS ANTI-FILAGGRINE DANS LE SERUM ET LE TISSU SYNOVIAL DE PATIENTS ATTEINTS DE POLYARTHRITE RMUMATOIDE

Du tissu synovial lésé a été prélevé au cours d'interventions chirurgicales chez 4 patients atteints de polyarthrite rhumatoïde, et séparé de la capsule et du cartilage articulaire. Un extrait de ce tissu a été préparé comme suit :

Des fragments de tissu synovial ont été homogénéisés (5 séquences d'homogénéisation) pendant 20 à 30 secondes à 0°C, avec un homogénéiseur ULTRA-TURRAX (JANKE & KUNKEL, Staufen, Allemagne) à la vitesse la plus élevée, dans 3 ml/g de tissu du tampon suivant : 40 mM Tris-HCl, pH 7,4, contenant 150 mM NaCl, 10 mM EDTA, 0,02% nitrate de sodium, 2 µg/ml d'Aprotinine (SIGMA) et 1 mM de PMSF.

Après chaque extraction, les homogénats ont été centrifugés à 4°C, pendant 20 minutes, à 15 000 g, et les surnageants, ont été prélevés, puis conservés à -80°C, jusqu'aux analyses. Les concentrations en protéines ont été déterminées en utilisant le test Coomassie Plus (PIERCE CHEMICALS, Rockford, IL).

Les concentrations en IgG totales et en anticorps anti-filaggrine de l'extrait de tissu synovial obtenu pour chacun des 4 patients ont été déterminés par ELISA, comme décrit à l'exemple 1 ci-dessus. L'extrait de tissu synovial est utilisé à une dilution de 1/400 à 1/32000, pour le dosage des IgG totales, et à une dilution de 1/2 à 1/20 pour le dosage des anticorps anti-filaggrine.

Ces concentrations en IgG totales et en anticorps anti-filaggrine ont été comparées avec celles déterminées dans les sérums des mêmes patients.

Les résultats sont illustrés par le tableau I ci-dessous.

**TABLEAU I**

| Patient | Sérum | | | Extrait synovial | | | Proportion relative d'AAF |
|---|---|---|---|---|---|---|---|
| | IgG (mg/ml) | AAF (µg/ml) | AAF/IgG (%) | IgG (mg/g) | AAF (µg/g) | AAF/IgG (%) | |
| 1 | 10,5 | 33,5 | 0,32 | 1,5 | 53,2 | 3,65 | 11,5 |
| 2 | 10,8 | 41,3 | 0,38 | 1,2 | 28,6 | 2,36 | 6,1 |
| 3 | 5,6 | 42,3 | 0,76 | 2,4 | 67,7 | 2,85 | 3,7 |
| 4 | 14,6 | 90,0 | 0,62 | 4,7 | 256,2 | 5,39 | 8,7 |
| Moyenne | 10,4±1,9 | 51,8±12,9 | 0,52±0,10 | 2,4±0,8 | 101,4±52,2 | 3,56±0,67 | 7,5±1,7 |

Ces résultats montrent que dans les sérums, la concentration moyenne en IgG totales est de 10,4 ± 1,9 mg/ml, ce qui correspond à la valeur physiologique normale de la concentration sérique en IgG, à l'exception d'un seul patient (patient 3) qui a une faible concentration sérique en IgG totales (5,6 mg/ml). La concentration moyenne en anticorps anti-filaggrine est de 51,8±12,9 µg/ml. Les anticorps anti-filaggrine correspondent donc en moyenne à 0,52 ± 0,1% des IgG totales, le patient 3 s'écartant peu de cette moyenne.

Dans les extraits synoviaux, les concentrations en anticorps sont exprimés en mg d'IgG totales ou en µg d'anticorps anti-filaggrine, par gramme de tissu synovial. La concentration moyenne synoviale en IgG totales est 5 fois plus faible que celle mesurée dans les sérums (2,4 ± 0,8 mg/g). En revanche, la concentration moyenne en anticorps anti-filaggrine dans le tissu synovial est 2 fois plus élevée que celle mesurée dans les sérums : 101,4 ± 52,2 µg/g. En conséquence, le rapport moyen AAF/IgG est de 3,56 ± 0,67% dans le tissu synovial contre 0,52 ± 0,10% dans le sérum.

Il est ainsi possible de calculer la proportion relative moyenne d'anticorps anti-filaggrine dans le tissu synovial, qui est égal au rapport des 2 rapports AAF/IgG dans les extraits synoviaux et dans le sérum respectivement. Cette proportion moyenne relative d'AAF est de 7,5 ± 1,7% ; en d'autres termes, pour les 4 patients, la quantité moyenne d'anticorps anti-filaggrine dans le tissu synovial est 7 fois et demie supérieure à celle mesurée dans le sérum.

### EXEMPLE 3 : DETECTION HISTOLOGIQUE DES PLASMOCYTES DANS LE TISSU SYNOVIAL.

Des échantillons de 5 mm³ de tissu synovial ont été préparés, et fixés pendant 24 heures à température ambiante dans une solution à 10% de formol salin. Ces fragments ont ensuite été déshydratés à l'alcool et inclus dans la paraffine. Des coupes de 4 microns d'épaisseur ont été effectuées, et colorées à l'hématoxyline/éosine. Ces coupes colorées ont été examinées au microscope.

Cette observation fait apparaître une hyperplasie de la couche de bordure de la synoviale, et une infiltration importante par des cellules mononucléées, disposées en agrégats nodulaires, autour de centres germinatifs (follicules lymphoïdes). Les plasmocytes ont été identifiés par leur noyau excentré contenant une chromatine en amas, et entouré par un cytoplasme abondant. La plupart des cellules étaient localisées à proximité immédiate des vaisseaux sanguins, ainsi que mélangées aux cellules synoviales de bordure, et entourant les follicules lymphoïdes. Les plasmocytes apparaissent comme étant le type cellulaire prédominant dans les infiltrats mononucléés des villosités synoviales, et ils constituent une proportion significative de la population de cellules mononucléées dans la plupart des échantillons examinés. Le nombre de cellules mononucléées par champ examiné varie de 580 à 560, et celui des plasmocytes de 68 à 280 ; ces derniers constituent donc 10 à 50% environ de la population totale de cellules mononucléées.

### Observations en immunofluorescence

Des échantillons de tissu synovial ont également été préparés pour l'observation en immunofluorescence. Des fragments de tissu, congelés dans l'isopentane préalablement refroidi par l'azote liquide sont conservés à -80°C jusqu'à l'observation. Les fragments congelés sont inclus dans du milieu TISSUE-TEK (REICHERT-JUNG, Heidelberg, Allemagne) et des coupes de 4 microns d'épaisseur sont effectuées dans un cryostat et montées sur lames de verre. Les coupes sont séchées à l'air pendant 10 minutes et conservées à -80°C jusqu'aux analyses immunohistochimiques.

Pour l'immunofluorescence, les coupes sont d'abord hydratées pendant 15 minutes dans du PBS, puis incubées pendant 30 minutes à 37°C en chambre humide, avec une dilution au 1/50 dans du PBS de fragments Fab de chèvre dirigés contre la chaîne lourde γ des IgG humaines, marqués à la fluorescéine. Après un rinçage dans du PBS contenant 0,05% de TWEEN-20, suivi d'un rinçage dans du PBS seul, les lames sont montées avec du milieu FLUOPREP (BIOMÉRIEUX, Lyon, France). L'observation au microscope, avec épi-illumination ultra-violette, montre une fluorescence intense de l'ensemble du tissu synovial, reflétant la présence des IgG interstitielles infiltrant celui-ci.

### EXEMPLE 4 : SYNTHÈSE ET SÉCRÉTION IN VITRO D'ANTICORPS ANTIFILAGGRINE PAR LES PLASMOCYTES SYNOVIAUX

Pour déterminer si des anticorps anti-filaggrine étaient produits par les plasmocytes synoviaux, des cultures du tissu synovial de patients atteints de polyarthrite rhumatoïde ont été effectuées comme suit : le tissu synovial frais a été partagé en petits fragments d'environ 1 à 2 mm de diamètre, immédiatement transférés dans des plaques de culture à 24 puits (un fragment par puits), remplis avec 2 ml de RPMI 1640-GLUTAMAX, supplémenté avec 10% de sérum de veau foetal, et 50 µg/ml de gentamycine (LIFE TECHNOLOGIES).

Les plaques de culture ont été incubées à 37°C sous atmosphère à 5% de CO₂ et 95% d'humidité.

Pour mesurer la production *in vitro* des IgG totales et des anticorps anti-filaggrine, le tissu synovial a été maintenu en culture pendant 34 jours. La totalité du milieu de culture a été changée le 1er jour, le 6^{ème} jour, le 13^{ème} jour, le 20^{ème} jour et le 27^{ème} jour de culture. Les surnageants de culture correspondants ont été conservés à -30°C jusqu'à analyse.

Les concentrations en IgG totales et en anticorps anti-filaggrine dans ces surnageants de culture ont été mesurées par ELISA, comme décrit à l'exemple 1 ci-dessus. Les surnageants de culture ont été utilisés à une dilution de 1/100 pour le dosage des IgG totales, et à une dilution de 1/10 pour le dosage des anticorps anti-filaggrine.

Afin d'évaluer le relargage des immunoglobulines infiltrant le tissu synovial, des coupes effectuées après 24 heures de culture, ont été préparées et observées par immunofluorescence comme décrit à l'exemple 4 ci-dessus. La fluorescence d'ensemble du tissu synovial a disparu, ce qui témoigne du relargage des immunoglobulines qui l'infiltraient ; en revanche, on observe des cellules de morphologie plasmocytaire groupées en amas, et présentant une fluorescence intracytoplasmique, témoignant de leur nature plasmocytaire, et de la production d'immunoglobulines par ces cellules.

Dans la mesure où ces observations par immunofluorescence montrent que les IgG relarguées pendant le 1^{er} jour de culture correspondent pour la plupart aux anticorps infiltrant le tissu, les concentrations en anticorps anti-filaggrine et en IgG mesurées dans les surnageants de culture du jour 0 et du jour 1 n'ont pas été prises en compte pour le calcul des concentrations cumulées produites pendant la totalité de la période de culture.

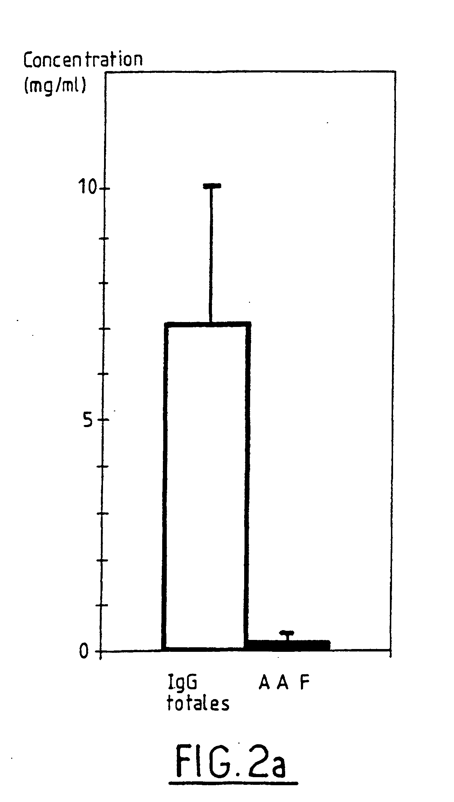

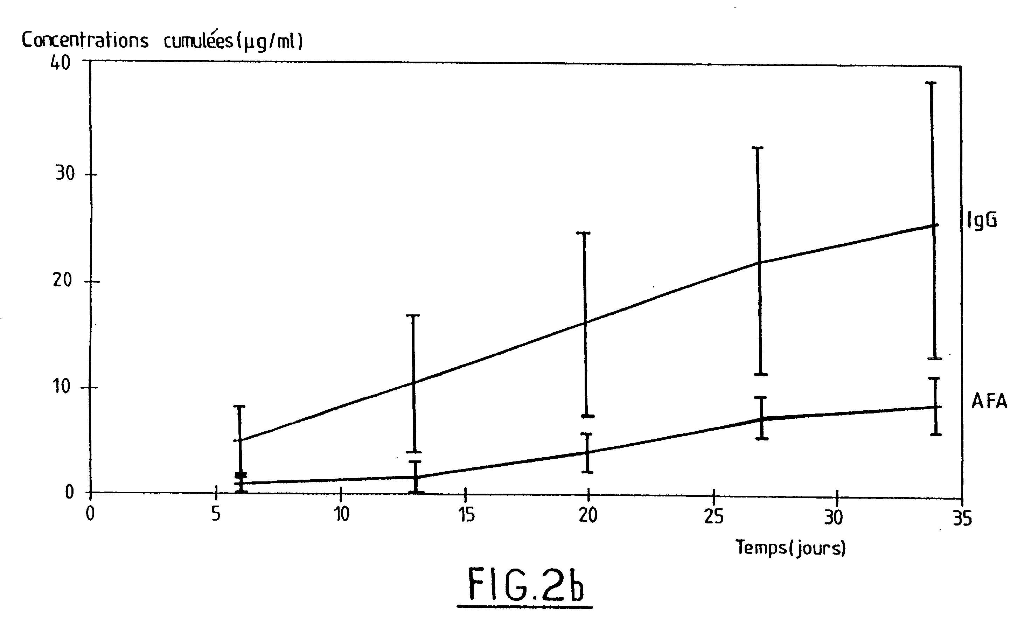

Les concentrations d'anticorps anti-filaggrine ou d'IgG déterminées pour le patient n°3 sont illustrées par la Figure 2.

La figure 2a représente les concentrations en anticorps anti-filaggrine (■) et en IgG (□) mesurées dans les surnageants de culture au jour 0 et au jour 1. Les anticorps anti-filaggrine correspondent à 2,8% des IgG totales dans le surnageant de culture des jours 0 et 1, ce qui correspond aux résultats obtenus avec les extraits de tissu synovial du même patient.

La figure 2b représente les concentrations d'anticorps anti-filaggrine (courbe du bas) et d'IgG (courbe du haut) produites au cours de la période de culture.

On constate que la production des IgG et des AAF est faible au 6^{ème} jour, puis augmente et atteint un pic au 27^{ème} jour. Ce profil de production ne peut s'expliquer que par une synthèse « *de novo* » d'IgG qui s'infléchit à partir du 27^{ème} jour.

En fin de culture, les anticorps anti-filaggrine représentent 34% des IgG totales.

Ces résultats montrent que les anticorps anti-filaggrine représentent une proportion très élevée des IgG totales sécrétées par le pannus rhumatoïde.

### EXEMPLE 5 : PROPRIÉTÉS INHIBITRICES DES PEPTIDES COMPRENANT LE MOTIF SER-CIT-HIS, SUR LA RÉACTION DES SÉRUMS DE PATIENTS ATTEINTS DE PR VIS-À-VIS DE FILAGGRINES CITRULLINÉES

Deux préparations d'antigène, à savoir : de la filaggrine recombinante citrullinée par déimination (obtenue selon le protocole décrit dans la demande PCT/FR97/01541), ou une préparation partiellement purifiée de filaggrine acido/neutre humaine (obtenue selon le protocole décrit par SIMON et al., J. Clin. Invest., 92, 1387, 1993) ont été déposées (0,2 µg par dépôt) sur un gel d'électrophorèse (gel PHAST®-SDS PHARMACIA) ; après électrophorèse avec l'appareil PHAST-SYSTEM® (PHARMACIA), dans les conditions préconisées par le fabricant, les protéines ont été transférées sur nitrocellulose. Les membranes de nitrocellulose ont été incubées (1 nuit, 4°C) avec un sérum (dilué au 1/4000^{ème}) d'un patient atteint de PR, et en présence de 40 µl/ml de chacun des peptides E12Hcit et E12Dcit (respectivement dérivés des peptides SEQ ID NO:2 ou SEQ ID NO:3 par remplacement de l'arginine centrale du motif Ser-Arg-His par une citrulline), ou d'un mélange de ces peptides. Pour chaque antigène, 2 sérums présentant un titre élevé en anticorps anti-filaggrine, et provenant de 2 patients différents ont été testés

A titre de contrôle, des incubations ont également été réalisées :
- en présence d'un peptide citrulliné (T12Ecit) dérivé du peptide SEQ ID NO:4, qui ne contient pas le motif Ser-Arg-His ;
- en l'absence de tout peptide.

Pour chaque essai, le produit de la réaction entre les anticorps anti-filaggrine du sérum du patient et l'antigène déposé sur la nitrocellulose a été détecté par l'intermédiaire d'anticorps de chèvre anti IgG humaines, marqués à la peroxydase.

Les résultats sont les suivants :
- pour chacun des 2 sérums PR testés, on observe un marquage intense lorsque l'incubation a été effectuée en l'absence de peptide, ou en présence du peptide T12Ecit. Lorsque l'incubation a été effectuée en présence du peptide E12Hcit ou E12Dcit, on n'observe qu'un faible marquage, dans le cas de l'un des sérums testés, et aucun marquage dans le cas de l'autre sérum.

En outre, lorsque l'incubation a été effectuée en présence d'un mélange des peptides E12Dcit et E12Hcit, on n'observe aucun marquage, quel que soit le sérum PR concerné.

Les résultats observés sont identiques pour les 2 antigènes (filaggrine recombinante déiminée ou préparation de filaggrine acido/neutre) testés.

Ces résultats montrent que les peptides E12Dcit et E12Hcit comprenant le motif Ser-Cit-His, sont capables d'inhiber, de manière compétitive, la réaction des anticorps « anti-filaggrine » présents dans le sérum des patients atteints de PR, avec les épitopes reconnus par ces anticorps.

### LISTE DE SEQUENCES

<110> UNIVERSITE PAUL SABATIER/TOULOUSE III
   SERRE, Guy
   GIRBAL-NEUHAUSER, Elisabeth
   VINCENT, Christian
   SIMON, Michel
   SEBBAG, Mireille
   DALBON, Pascal
   JOLIVET-REYNAUD, Colette
   ARNAUD, Michel
   JOLIVET, Michel
<120> UTILISATION DE PEPTIDES CITRULLINES DERIVES DE LA FILAGGRINE DANS LE CADRE DU TRAITEMENT DE MALADIES AUTOIMMUNES
<130> MJPcb1249/1
<140>
<141>
<150> FR9716672
<151> 1997-12-30
<160> 4
<170> PatentIn Ver. 2.1
<210> 1
<211> 49
<212>
<213> Homo sapiens
<400> 1 STGHSGSQHS HTTTQGRSDA SRGSSGSRST SRETRDQEQS GDGSRHSGS 49
<210> 2
<211> 14
<212>
<213> Homo sapiens
<400> 2 eqsadssrhs gsgh 14
<210> 3
<211> 14
<212>
<213> Homo sapiens
<400> 3 essrdgsrhp rshd 14
<210> 4
<211> 14
<212>
<213> Homo sapiens
<400> 4

tgsstggrqg shhe 14

## Revendications

1. Utilisation d'un antigène peptidique reconnu spécifiquement par les auto-anticorps anti-filaggrine présents dans le sérum de patients atteints de polyarthrite rhumatoïde et constitué par un peptide comprenant le motif tripeptidique Ser-Cit-His dans lequel Cit représente un résidu citrulline, lequel peptide est dérivé de tout ou partie de la séquence d'une unité filaggrine par substitution d'au moins un résidu arginine par un résidu citrulline, pour l'obtention d'un médicament destiné au traitement d'une pathologie impliquant une réponse autoimmune vis-à-vis d'épitopes citrullinés reconnus par lesdits anticorps anti-filaggrine, à l'exception de l'utilisation d'un peptide de séquence : Gln Gly Ser Cit His Gln Gln Ala Arg et des peptides comprenant ladite séquence, dérivés de l'un des peptides suivants :
- Gly Gly Gln Gly Ser Arg His Gln Gln Ala Arg ;
- Gln Gly Ser Arg His Gln Gln Ala Arg Asp Ser Ser Arg His Ser Thr Ser Gln Glu Gly Gln Asp Thr Ile His Gly His Arg Gly Ser ;
- Gln Gly Ser Arg His Gln Gln Ala Arg Asp Ser Ser Arg His Ser Ala Ser Gln Asp Gly Gln Asp Thr Ile Arg Gly His Pro Gly Ser Ser ;
par substitution d'au moins un résidu arginine par un résidu citrulline.

2. Utilisation selon la revendication 1, **caractérisée en ce que** ledit antigène est constitué par un peptide comprenant tout ou partie d'au moins une séquence dérivée :
- de la séquence correspondant aux acides aminés 144 à 324 d'une unité filaggrine humaine, ou bien
- de la séquence correspondant aux acides aminés 76 à 144 d'une unité filaggrine humaine, ou bien
- de la séquence correspondant aux acides aminés 71 à 119 d'une unité de filaggrine humaine,
par substitution d'au moins un résidu arginine par un résidu citrulline.

3. Utilisation selon la revendication 1, **caractérisée en ce que** ledit antigène est constitué par un peptide comprenant tout ou partie d'au moins une séquence dérivée de l'une des séquences SEQ ID NO: 1, SEQ ID NO: 2, SEQ ID NO: 3, par substitution d'au moins un résidu arginine par un résidu citrulline.

4. Utilisation selon une quelconque des revendications 1 à 3, **caractérisée en ce que** ledit peptide comprend le motif pentapeptidique, X1-Ser-Cit-His-X2 dans lequel Cit représente un résidu citrulline, X1 représente un résidu sérine ou glycine, et X2 représente un résidu sérine ou proline.

5. Composition pharmaceutique pour le traitement de la polyarthrite rhumatoïde, **caractérisée en ce qu'**elle contient en tant que principe actif, au moins un peptide antigénique tel que défini dans une quelconque des revendications 1 à 3,
à l'exception de l'utilisation d'un peptide de séquence : Gln Gly Ser Cit His Gln Gln Ala Arg et des peptides comprenant ladite séquence, dérivés de l'un des peptides suivants :
- Gly Gly Gln Gly Ser Arg His Gln Gln Ala Arg ;
- Gin Gly Ser Arg His Gln Gln Ala Arg Asp Ser Ser Arg His Ser Thr Ser Gln Glu Gly Gln Asp Thr Ile His Gly His Arg Gly Ser ;
- Gln Gly Ser Arg His Gln Gln Ala Arg Asp Ser Ser Arg His Ser Ala Ser Gln Asp Gly Gln Asp Thr Ile Arg Gly His Pro Gly Ser Ser ;
par substitution d'au moins un résidu arginine par un résidu citrulline.

## Claims

1. The use of a peptide antigen specifically recognised by the anti-filaggrin autoantibodies present in the serum of patients affected by rheumatoid arthritis and consisting of a peptide containing the Ser-Cit-His tripeptide motif in which Cit represents a citrulline residue, the peptide being derived from all or part of the sequence of a filaggrin unit as a result of the substitution of at least one arginine residue with a citrulline residue for the purposes of obtaining a drug intended for the treatment of a pathology involving an autoimmune response vis-à-vis citrullinated epitopes recognised by the said anti-filaggrin autibodies, with the exception of the use of a peptide with the sequence: Gln Gly Ser Cit His Gln Gln Ala Arg and peptides containing this sequence which are derived from one of the following peptides:
- Gly Gly Gln Gly Ser Arg His Gln Gln Ala Arg;
- Gln Gly Ser Arg His Gln Gln Ala Arg Asp Ser Ser Arg His Ser Thr Ser Gln Glu Gly Gln Asp Thr Ile His Gly His Arg Gly Ser
- Gln Gly Ser Arg His Gln Gln Ala Arg Asp Ser Ser Arg His Ser Ala Ser Gln Asp Gly Gin Asp Thr Ile Arg Gly His Pro Gly Ser Ser;
as a result of the substitution of at least one arginine residue with a citrulline residue.

2. Use according to claim 1, **characterised by** the fact that the said antigen consists of a peptide which includes all or part of at least one sequence derived:
- from the sequence corresponding to amino acids 144 to 324 of a human filaggrin unit or
- from the sequence corresponding to amino acids 76 to 144 of a human filaggrin unit or
- from the sequence corresponding to amino acids 71 to 119 of a human filaggrin unit as a result of the substitution of at least one arginine residue with a citrulline residue.

3. Use according to claim 1, **characterised by** the fact that the said antigen consists of a peptide which includes all or part of at least one sequence derived from one of the sequences SEQ ID NO: 1, SEQ ID NO: 2 or SEQ ID NO: 3 as a result of the substitution of at least one arginine residue with a citrulline residue.

4. Use according to any of claims 1 to 3, **characterised by** the fact that the said peptide contains the pentapeptide motif, X1-Ser-Cit-His-X2, in which Cit represents a citrulline residue, X1 represents a serine or glycine residue, and X2 represents a serine or proline residue.

5. Pharmaceutical compound for the treatment of rheumatoid arthritis, **characterised by** the fact that it contains as the active substance at least one antigenic peptide such as defined in any of claims 1 to 3, with the exception of the use of a peptide with the sequence: Gln Gly Ser Cit His Gln Gln Ala Arg; and peptides containing this sequence which are derived from one of the following peptides:
- Gly Gly Gln Gly Ser Arg His Gln Gln Ala Arg;
- Gln Gly Ser Arg His Gln Gln Ala Arg Asp Ser Ser Arg His Ser Thr Ser Gln Glu Gly Gln Asp Thr Ile His Gly His Arg Gly Ser;
- Gln Gly Ser Arg His Gln Gln Ala Arg Asp Ser Ser Arg His Ser Ala Ser Gln Asp Gly Gln Asp Thr Ile Arg Gly His Pro Gly Ser Ser;
as a result of the substitution of at least one arginine residue with a citrulline residue.

## Patentansprüche

1. Verwendung eines peptidischen Antigens, das spezifisch von den anti-Filaggrin-Autoantikörpern im Serum von Patienten mit rheumatoider Polyarthritis erkannt wird und aus einem Peptid besteht, das das Tripeptid-Motiv Ser-Cit-His umfaßt, in dem Cit einen Citrullinrest bedeutet, wobei sich das Peptid ganz oder teilweise von der Sequenz einer Filaggrin-Einheit durch Substitution wenigstens eines Argininrests durch einen Citrullinrest ableitet, zur Herstellung eines Medikaments zur Behandlung einer Pathologie, die eine Autoimmunreaktion gegenüber Citrullin-Epitopen umfaßt, die von diesen anti-Filaggrin-Antikörpern erkannt werden, ausgenommen die Verwendung eines Peptids mit der Sequenz: Gln Gly Ser Cit His Gln Gln Ala Arg und von Peptiden, die diese Sequenz enthalten, abgeleitet von einem der folgenden Peptide:
- Gly Gly Gln Gly Ser Arg His Gln Gln Ala Arg;
- Gln Gly Ser Arg His Gln Gln Ala Arg Asp Ser Ser Arg His Ser Thr Ser Gln Glu Gly Gln Asp Thr Ile His Gly His Arg Gly Ser;
- Gln Gly Ser Arg His Gln Gln Ala Arg Asp Ser Ser Arg His Ser Ala Ser Gln Asp Gly Gln Asp Thr Ile Arg Gly His Pro Gly Ser Ser;
durch Substitution wenigstens eines Argininrests durch einen Citrullinrest.

2. Verwendung nach Anspruch 1, **dadurch gekennzeichnet, daß** das Antigen aus einem Peptid besteht, das ganz oder teilweise wenigstens eine Sequenz umfaßt, die sich
- von der Sequenz, die den Aminosäuren 144 bis 324 einer Einheit von humanem Filaggrin entspricht, oder
- von der Sequenz, die den Aminosäuren 76 bis 144 einer Einheit von humanem Filaggrin entspricht, oder
- von der Sequenz, die den Aminosäuren 71 bis 119 einer Einheit von humanem Filaggrin entspricht,
durch Substitution wenigstens eines Argininrests durch einen Citrullinrest ableitet.

3. Verwendung nach Anspruch 1, **dadurch gekennzeichnet, daß** das Antigen aus einem Peptid besteht, das ganz oder teilweise wenigstens eine Sequenz umfaßt, die sich von einer der Sequenzen SEQ ID NO: 1, SEQ ID NO: 2, SEQ ID NO: 3 durch Substitution wenigstens eines Argininrests durch einen Citrullinrest ableitet.

4. Verwendung nach irgendeinem der Ansprüche 1 bis 3, **dadurch gekennzeichnet, daß** das Peptid das Pentapeptid-Motiv X1-Ser-Cit-His-X2 umfaßt, in dem Cit einen Citrullinrest bedeutet, X1 einen Serinoder Glycinrest bedeutet und X2 einen Serin- oder Prolinrest bedeutet.

5. Pharmazeutische Zusammensetzung zur Behandlung von rheumatoider Polyarthritis, **dadurch gekennzeichnet, daß** sie als Wirkstoff wenigstens ein wie in irgendeinem der Ansprüche 1 bis 3 definiertes antigenes Peptid enthält,
ausgenommen die Verwendung eines Peptids der Sequenz: Gln Gly Ser Cit His Gln Gln Ala Arg und von Peptiden, die diese Sequenz enthalten, abgeleitet von einem der folgenden Peptide:
- Gly Gly Gln Gly Ser Arg His Gln Gln Ala Arg;
- Gln Gly Ser Arg His Gln Gln Ala Arg Asp Ser Ser Arg His Ser Thr Ser Gln Glu Gly Gln Asp Thr Ile His Gly His Arg Gly Ser;
- Gln Gly Ser Arg His Gln Gln Ala Arg Asp Ser Ser Arg His Ser Ala Ser Gln Asp Gly Gln Asp Thr Ile Arg Gly His Pro Gly Ser Ser;
durch Substitution wenigstens eines Argininrests durch einen Citrullinrest.
